# EUROPEAN PATENT SPECIFICATION

(11) **EP 2 240 071 B1**
(45) Date of publication and mention of the grant of the patent: **15.10.2014**
(21) Application number: 08863138.7
(22) Date of filing: 15.12.2008
(51) Int. Cl.: A61B 5/1455, A61B 5/00

(54) **MEDICAL SENSOR AND METHOD OF MANUFACTURING THE SAME**
MEDIZINISCHER SENSOR UND VERFAHREN ZU SEINER HERSTELLUNG
CAPTEUR MÉDICAL ET TECHNIQUE DE PRODUCTION DE CELUI-CI

(30) Priority: 14.12.2007 US 13850
(43) Date of publication of application: 20.10.2010
(73) Proprietor: Covidien LP, Mansfield, MA 02048 (US)
(72) Inventor: EGHBAL, Darius, Oakland California 94609 (US); HOARAU, Carine, Lafayette California 94549 (US)
(74) Representative: Hargreaves, Timothy Edward
(86) International application number: PCT/US2008/086846
(87) International publication number: WO 2009/079461

(56) References cited:
- EP-A- 0 357 249
- WO-A-2007/019420
- WO-A-2007/041333
- US-A1- 2006 173 247
- US-A1- 2007 021 660

## Description

### Technical Field

The present disclosure relates generally to medical devices and, more particularly, to sensors used for sensing physiological parameters of a patient.

### Background

This section is intended to introduce the reader to various aspects of art that may be related to various aspects of disclosed embodiments, which are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

In the field of medicine, doctors often desire to monitor certain physiological characteristics of their patients. Accordingly, a wide variety of devices have been developed for monitoring physiological characteristics. Such devices provide doctors and other healthcare personnel with the information they need to provide the best possible healthcare for their patients. As a result, such monitoring devices have become an indispensable part of modern medicine.

One such monitoring technique is commonly referred to as pulse oximetry, and the devices built based upon pulse oximetry techniques are commonly referred to as pulse oximeters. Pulse oximetry may be used to measure various blood flow characteristics, such as the blood-oxygen saturation of hemoglobin in arterial blood, the volume of individual blood pulsations supplying the tissue, and/or the rate of blood pulsations corresponding to each heartbeat of a patient.

Pulse oximeters typically utilize a non-invasive sensor that is placed on, adjacent, and/or against a patient's tissue that is well perfused with blood, such as a patient's finger, toe, forehead or earlobe, and/or other location. The pulse oximeter sensor may emit light, and photoelectrically sense the absorption and/or scattering of the light after passage through the perfused tissue. The data collected by the sensor may then be used to calculate one or more of the above physiological characteristics based upon the absorption or scattering of the light.

Interferences in the light path may distort the emitted and sensed light utilized in calculating the physiological characteristics. In particular, pulse oximetry may be sensitive to movement, such as moving the sensor in relation to the tissue, increasing or decreasing the physical distance between the emitter and detector in the sensor, changing the orientation of the emitter or detector with respect to the tissue or each other, changing the angles of incidence and interfaces probed by the light, directing the optical path through different amounts or types of tissue, and expanding, compressing, or otherwise altering the tissue near the sensor.

WO 2007/019420 describes a sensor for pulse oximetry measurements that includes diaphragm structures that deform and spread force over a large area when attached to a patient's digit, thereby to reduce pressure on the digit. WO 2007/041333 describes a clip-style sensor for pulse oximetry measurements. US 2006/0173247 describes a finger sleeve sensor holder for pulse oximetry measurements that is both plastically and elastically deformable. EP 0 357 249 describes a finger sensor for a pulse oximetry system that comprises a molded polymeric body.

US 2007/0260129 describes a sensor for pulse oximetry that comprises a gripping region that contacts the patient's skin. US 2007/0073121 describes a sensor for pulse oximetry. The sensor may be a flexible sensor provided with a stiffening member, an annular or partially annular sensor, or a clip-style sensor including a spacer. US 2007/0078317 describes a sensor assembly for pulse oximetry measurements that includes a frame and an overmold coating provided about the frame. US 2007/0078309 describes a physiological sensor that includes an emitter and a detector disposed on a frame, wherein the frame includes one or more pairs of flexible elements. US 2007/0021660 describes a sensor for pulse oximetry that includes an emitter, a detector and a shunt barrier between the emitter and the detector to block shunted light from the emitter.

### SUMMARY

The invention is defined in independent claims 1 and 17 and the dependent claims 2-16.

According to an embodiment, there is provided a sensor, including an emitter and a detector at a substantially fixed optical distance relative to one another, and an expandable molded body for securing secure the emitter and the detector to a digit.

According to an embodiment, there is provided a sensor body, including a semi-rigid molded structure having a generally tubular interior and a lengthwise opening to enable expansion of the generally tubular interior to accommodate a digit.

According to an embodiment, there may be still further provided a sensor body, including a flexible semi-rigid structure configured to be affixed to sensing components and to secure the sensing components to a digit.

According to an embodiment, there is further provided a method of manufacturing a sensor, including overmolding a flexible semi-rigid sensor body around an emitter and a detector.

Finally, according to an example, there may be provided a method of manufacturing a sensor, including enclosing an emitter and a detector in a substantially rigid casing, molding a flexible sensor body, and coupling the substantially rigid casing to the flexible sensor body.

### BRIEF DESCRIPTION OF THE DRAWINGS

Advantages of this disclosure may become apparent upon reading the following detailed description and upon reference to the drawings in which:
FIG. 1 is a perspective view of an exemplary patient monitoring system, in accordance with aspects of an embodiment;
FIG. 2 is a perspective view of an exemplary sensor for use with the patient monitoring system of FIG. 1, in accordance with aspects of an embodiment;
FIG. 3 is a perspective view of the exemplary sensor of FIG. 2 applied to a patient's digit, in accordance with aspects of an embodiment;
FIG. 4 is an exploded view of the exemplary sensor of FIG. 2, in accordance with aspects of an embodiment;
FIG. 5 is a perspective view of another exemplary sensor for use with the patient monitoring system of FIG. 1, in accordance with aspects of an example;
FIG. 6 is a perspective view of a further exemplary sensor for use with the patient monitoring system of FIG. 1, in accordance with aspects of an example; and
FIG. 7 is a perspective view of another exemplary sensor for use with the patient monitoring system of FIG. 1, in accordance with aspects of an example.

### DERAILED DESCRIPTION

One or more specific embodiments and examples will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

In accordance with the present disclosure, sensors for pulse oximetry or other applications utilizing spectrophotometry may be provided that reduce signal artifacts by reducing the occurrence of tissue deformation, such as compression or discoloration, associated with the movement of a patient's tissue relative to the sensor or the movement of the sensor elements relative to one another. For example, sensors are provided herein that include a flexible elastomeric body that may accommodate patient movement.

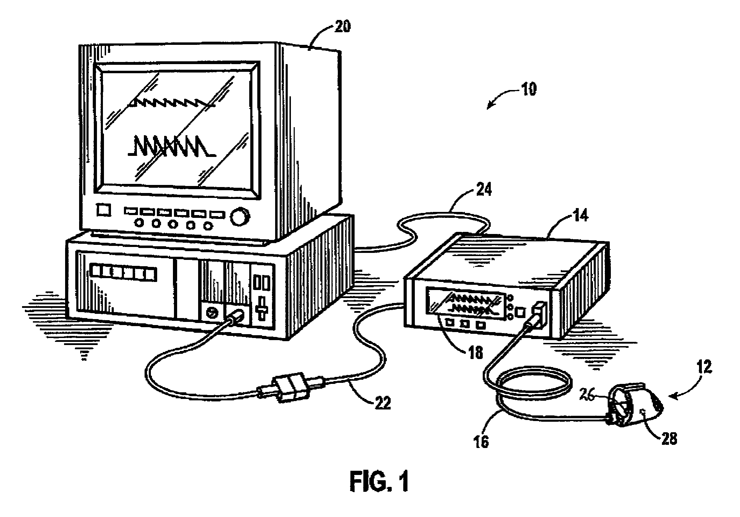

Prior to discussing such exemplary sensors in detail, it should be appreciated that such sensors may be designed for use with a typical patient monitoring system 10. For example, referring now to FIG. 1, a sensor 12 according to an embodiment may be used in conjunction with a monitor 14. In the depicted embodiment, a cable 16 connects the sensor 12 to the monitor 14. It should be appreciated that the sensor 12 and/or the cable 16 may include or incorporate one or more integrated circuit devices or electrical devices, such as a memory, processor chip, or resistor, that may facilitate or enhance communication between the sensor 12 and the monitor 14. Likewise the cable 16 may be an adaptor cable, with or without an integrated circuit or electrical device, for facilitating communication between the sensor 12 and various types of monitors, including older or newer versions of the monitor 14 or other physiological monitors. In other embodiments, the sensor 12 and the monitor 14 may communicate via wireless means, such as using radio, infrared, or optical signals. In such embodiments, a transmission device (not shown) may be connected to the sensor 12 to facilitate wireless transmission between the sensor 12 and the monitor 14. It should be appreciated that the cable 16 (or corresponding wireless transmissions) may be used to transmit control or timing signals from the monitor 14 to the sensor 12 and/or to transmit acquired data from the sensor 12 to the monitor 14. In another embodiment, the cable 16 may be an optical fiber that enables optical signals to be conducted between the monitor 14 and the sensor 12.

The monitor 14 may be a pulse oximeter, such as those available from Nellcor Puritan Bennett LLC, and/or Covidien, or may be a monitor for measuring other body related metrics using spectrophotometric and/or other techniques. Additionally, the monitor 14 may be a multi-purpose monitor suitable for performing pulse oximetry and measurement of tissue water fraction, or other combinations of physiological and/or biochemical monitoring processes, using data acquired via the sensor 12. Measured physiological parameters may be displayed on a display 18. Furthermore, the monitor 14 may be coupled to a multi-parameter monitor 20 via a cable 22 connected to a sensor input port and/or via a cable 24 connected to a digital communication port and/or other methods or configurations.

In an embodiment, the sensor 12 includes an emitter 26 and a detector 28 which may be of any suitable type. For example, the emitter 26 may be one or more light emitting diodes adapted to transmit one or more wavelengths of light, such as in the red to infrared range, and the detector 28 may be a photodetector, such as a silicon photodiode package, selected to receive light in the range emitted from the emitter 26. The sensor 12 may transmit electrical and/or optical signals to and from the monitor 14 via the cable 16. The cable 16 may be permanently or removably coupled to the sensor 12, the latter alternative may be more useful and cost efficient in situations where the sensor 12 is disposable.

Pulse oximetry and other spectrophotometric sensors may be typically placed on a patient in a location conducive to measurement of the desired physiological parameters. For example, pulse oximetry sensors may be typically placed on a patient in a location that is normally perfused with arterial blood to facilitate measurement of the desired blood characteristics, such as blood oxygen saturation (SpO₂). Common pulse oximetry sensor sites may include, but is not limited to, a patient's fingertips, toes, forehead, or earlobes. Regardless of the placement of the sensor 12, the reliability of the pulse oximetry measurement may be related to the accurate detection of transmitted light that has passed through the perfused tissue and has not been inappropriately supplemented by outside light sources or modulated by subdermal anatomic structures. Such inappropriate supplementation and/or modulation of the light transmitted by the sensor may cause variability in the resulting pulse oximetry measurements.

The sensor 12 described above may be generally configured for use as a "reflectance type" sensor for use in spectrophotometric applications. Reflectance type sensors include an emitter and detector that are typically placed on the same side of the sensor site. If the sensor site is a fingertip, for example, the sensor 12 may be placed on a patient's fingertip such that the emitter and detector are positioned on the same side of the finger. Reflectance type sensors detect light photons that are scattered back to the detector. During operation, the emitter emits one or more wavelengths of light into the patient's fingertip, or other tissue, and the light received by the detector is processed to determine various physiological characteristics of the patient.

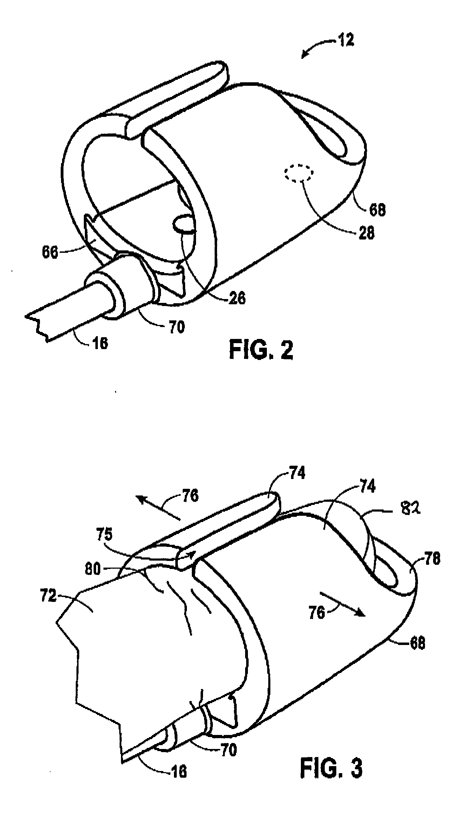

Referring now to FIG. 2, an exemplary embodiment of the sensor 12 includes a sensing component assembly 66 and a sensor body 68. The sensing component assembly 66 may include a rigid or semi-rigid casing configured to hold the emitter 26 and the detector 28 at a substantially fixed optical distance in relation to one another when the sensor 12 is applied to a patient. The emitter 26 and the detector 28 may be connected to the cable 16 by one or more wires 70. The wires 70 may be coupled to the cable 16 to enable transmission of data to and from the monitor 12 (FIG. 1).

In one embodiment, the sensor body 68 is a flexible, semi-rigid material capable of elastic deformation, or expansion. The sensor body 68 has a generally tubular shape which may be capable of conforming to a patient's digit 72, as illustrated in FIG. 3. One or more wings 74, are separated by a lengthwise opening 75, and are capable of securing the sensor 12 to the patient's digit 72. The wings 74 may be configured to expand laterally, indicated by arrows 76, to accommodate larger digits, swelling or edema, while remaining sufficiently positioned around the patient's digit 72 to ensure the sensing component assembly 66 remains generally flush with the patient's digit 72. In order to ensure that the wings 74 fit securely but comfortably around the patient's digit 72, multiple sizes of the sensor 12 may be manufactured. Additionally, to improve patient comfort, the cable 16 may have a relatively flat profile, such as a ribbon cable, and be disposed under the patient's digit 72.

The sensor body 68 may be composed of any suitable material such that the wings 74 are flexible enough to expand, yet rigid enough to secure the sensor 12 to the patient's digit 72. Additionally, the material used in the sensor body 68 may be considered semi-rigid in that, unlike a bandage sensor, the sensor body 68 may generally maintain its digit-conforming shape even when not secured to the patient's digit 72. In an embodiment, the sensor body 68 may be composed of an elastomer with a durometer of about 15-30 Shore A. In an embodiment, the sensor body 68 may be composed of a material that may be flexible and easily cleanable, such as polyurethane, santoprene, silicone thermoset, or any other medical grade elastomer, which may allow the sensor 12 to be reused. Alternatively, the sensor 12 and/or the sensor body 68 may be configured to be disposable, and the sensor body 68 may be composed of a closed cell foam elastomer or other suitable material. In addition, the sensor body 68 may be composed of a tack material or coated with an adhesive layer, either partially or fully, to aid in securing the sensor 12 to the patient's digit 72. Furthermore, one or more adhesive layers may be included, such that when one adhesive layer is reduced in its bonding abilities, it may be removed to expose another adhesive layer with relatively greater bonding characteristics.

Environmental light may contribute to errors in physiological measurements. Therefore, it may be desirable to prevent light from the environment from reaching the detector. This environmental light may enter the sensor through gaps between the sensor body and the patient's tissue. For example, in many clip-style sensors the clip may open partially and allow invasion of environmental light when the patient's finger is bent. The sensor body 68 may be configured to minimize interference of light from outside the sensor 12. The wings 74 may be configured to prevent light from entering the sides of the sensor 12, and a lip 78 may be configured to prevent light from entering the end of the sensor 12. The sensor body 68 may also be configured such that the sensor 12 covers only the end of the patient's digit 72. For example, the sensor 12 may extend from the tip of the patient's digit 72 to an area short of a first digit joint 80. In this embodiment, the sensing component assembly 66 may remain securely in place and generally flush with the tissue of the patient's digit 72 even when the digit joint 80 is bent. In addition, the sensor body 68 may be configured to enable a fingernail 82 to protrude from the sensor 12 over the lip 78.

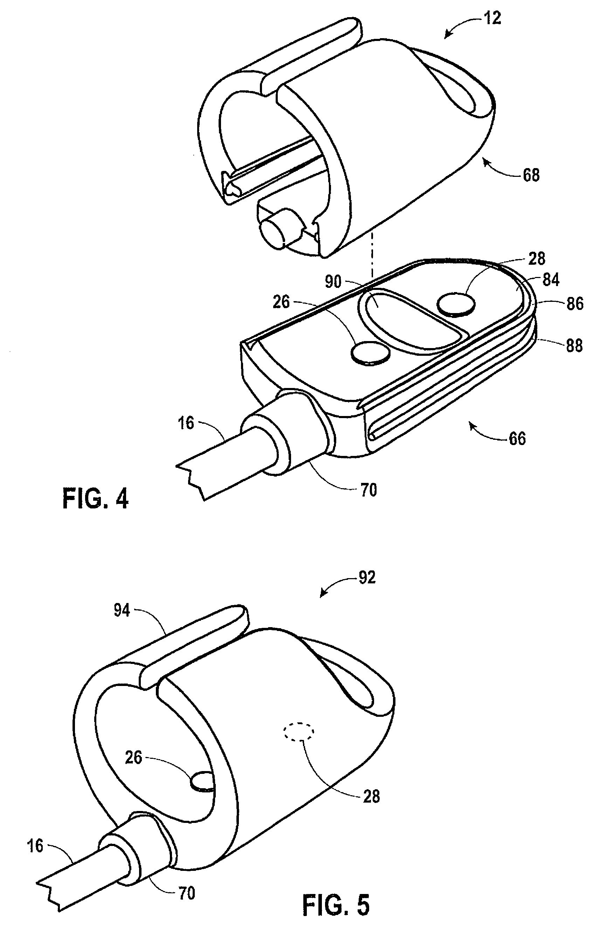

Turning now to FIG. 4, an exploded view of the sensor 12 is shown. The sensing component assembly 66 may include a casing 84 with a top 86, a bottom 88, and a lateral raised ridge 90. The sensing components, including the emitter 26, the detector 28, and any electrical components to enable their operation, may be enclosed in the casing 84. The casing 84 may be composed of any suitable rigid or semi-rigid material, such as a plastic or metal. By installing the emitter 26 and the detector 28 in a substantially rigid casing 84, they may be held at a substantially fixed optical position with respect to one another, thus limiting the modulations of detected light that may occur and the resulting measurement errors.

The casing 84 may be formed by any suitable means. In an embodiment, the top 86 and the bottom 88 may be formed separately by injection molding then coupled together to form the casing 84 around the sensing components. The top 86 and the bottom 88 may be configured such that they snap together to form the casing 84, or alternatively they may be coupled together using some form of adhesive. In another embodiment, the sensing components may be positioned within a die or mold and overmolded to form the casing 84. In addition, the casing 84 may be sufficiently water-tight that the sensor 12 and/or the sensing component assembly 66 may be washed and reused. The lateral raised ridge 90 may be configured to aid in proper positioning of the sensing component assembly 66 on the patient's digit 72 and to prevent the detector 28 from detecting scattered light from the emitter 26 that has not entered the patient's tissue.

The sensing component assembly 66 and the sensor body 68 may be coupled together by any suitable method. For example, according to one example, the sensing component assembly 66 and the sensor body 68 may be manufactured separately, then coupled together. For example, the sensor body 68 may be formed by injection molding then attached to the sensing component assembly 66 adhesively, such as via an adhesive layer, or mechanically, such as via slots and tabs. In an example, the sensing component assembly 66 and the sensor body 68 may be coupled together by the user. This method and configuration may be advantageous where the sensing component assembly 66 is capable of being reused and the sensor body 68 is configured to be disposable.

In an embodiment, the sensing component assembly 66 may be positioned within a die or mold, and overmolded to attach the sensor body 68. In this embodiment, to prevent separation of the sensor body 68 from the sensing component assembly 66, the material used to form the sensor body 68 may be selected such that the sensor body 68 bonds with the sensing component assembly 66 during the overmolding process. Techniques for protecting the sensing components from excessive temperatures may be employed. For example, the emitter 26 and/or the detector 28 may include respective translucent windows, such as a plastic or crystal window, adjacent the mold to prevent coating from being applied over the window. In an example, the material adjacent such windows may be composed of a material, such as beryllium copper, which prevents the heat of the injection molding process from being conveyed through the windows to the sensing components. For example, a beryllium copper material initially at about 40°F may be adjacent the windows associated with the emitter 26 and/or the detector 28 to prevent coating of the windows and heat transfer to the respective sensing components.

Another example of a sensor 92 for use with the patient monitoring system 10 of FIG. 1 is illustrated in FIG. 5. In this example, the sensor 92 may include a sensor body 94 that may be formed integrally with the sensing components, including the emitter 26, the detector 28, and any electrical components to enable their operation. For example, in an example, the sensing components may be positioned within a die or mold and overmolded with the material used to form the sensor body 94, thereby forming the entire sensor 92 of a flexible material. The sensor body 94 may be composed of any suitable material, as described above in reference to FIGS. 2-4. The sensor 92 may therefore be generally a unitary, expandable, semi-rigid molded device with no crevices in which unwanted particles may accumulate.

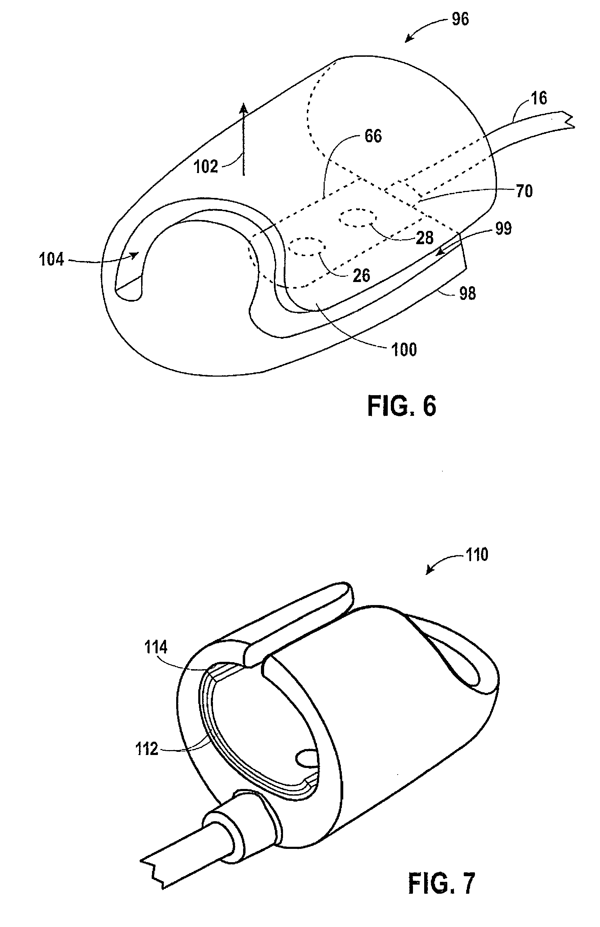

FIG. 6 illustrates another example of a sensor 96 for use with the patient monitoring system 10 of FIG. 1. In an example, the sensor body 98 may be generally tubular and have a lengthwise opening 99. A top flap 100 of the sensor body 98 may be configured to expand vertically, as indicated by an arrow 102, to accommodate larger digits, swelling or edema. The sensor body 98 may be composed of any suitable material, as described above in reference to FIGS. 2-4. In addition, the sensor body 98 may include an opening 104 to enable protrusion of the fingernail 82 from the patient's digit 72 (FIG. 3). As with the sensors 12 and 92 illustrated in FIGS. 2-5, examples of the sensor 96 may include a generally rigid or semi-rigid sensing component assembly and a separate sensor body, or the sensing components maybe integrated directly into the sensor body.

Further sensor improvements may also be incorporated. For example, FIG. 7 illustrates another example of a sensor 110 for use with the patient monitoring system 10 of FIG. 1. In this example, the sensor 110 includes a stack of adhesive layers 112 disposed on an interior surface 114. As discussed above, incorporation of an adhesive on the interior surface 114 of the sensor 110 may further secure the sensor 110 to the patient's digit 72 (FIG. 3). However, some adhesives may lose their adhesion after repented removal from and reapplication to a surface. Accordingly, the stack of adhesive layers 112 may allow the caregiver to remove the sensor 110, such as to check on the sensor site or move the patient, then reapply the sensor 110 with a fresh adhesive layer 112. That is, the top adhesive layer 112 may be removed to expose a fresh adhesive layer 112. Various embodiments of the adhesive layers 112 may be found in U.S. Patent No. 6,748,254, entitled "Stacked Adhesive Optical Sensor," and issued on June 8, 2004.

While the subject of this disclosure may be susceptible to various modifications and alternative forms, specific embodiments have been shown by way of example in the drawings and have been described in detail herein. However, it should be understood that this disclosure is not intended to be limited to the particular forms disclosed. Rather, the invention is to cover all modifications, equivalents, and alternatives falling within the scope of the invention as defined by the following appended claims. Indeed, the present techniques may not only be applied to reflectance type sensors for use in pulse oximetry, but also to retroflective and other sensor designs as well. Likewise, the present techniques are not limited to use on fingers and toes but may also be applied to placement on other body parts such as in embodiments configured for use on the cars or nose, or other locations.

## Claims

1. A sensor (12), comprising:
an emitter (26) and a detector (28), wherein the emitter (26) and the detector (28) are at a substantially fixed optical distance relative to one another; and
a generally expandable molded body (68) configured to secure the emitter (26) and the detector (28) to a digit, **characterised in that**:
the body (68) comprises a plurality of wings (74) and has a generally tubular shape and a generally tubular interior, wherein the wings (74) are separated by an opening extending generally lengthwise along the sensor;
the sensor further comprises a sensing component assembly (66) which is configured to be coupled to said to body (68) and comprises said emitter and detector and a casing (84) with a top (86), a bottom (88) and a lateral raised ridge (90) that is between the emitter (26) and the detector (28) and that is opposite the generally lengthwise opening when
the sensing component assembly (66) and the body (68) are coupled together.

2. The sensor of claim 1, wherein the expandable molded body (68) is overmolded onto the emitter (26) and the detector (28).

3. The sensor of claim 1, comprising a substantially rigid casing (84) configured to house the emitter (26) and the detector (28).

4. The sensor of claim 3, wherein the expandable molded body (68) is an overmolded component capable of being affixed to the substantially rigid casing (84).

5. The sensor of claim 3, wherein the expandable molded body (68) is a pre-molded component capable of being affixed to the substantially rigid casing (84), and optionally the pre-molded component is capable of being removably affixed to the substantially rigid casing (84).

6. The sensor of claim 1, wherein the expandable molded body (68) comprises an elastomer with a durometer of about 15 to about 30 Shore A.

7. The sensor of claim 1, wherein the expandable molded body (68) comprises a polyurethane, silicone thermoset, medical grade elastomer, closed cell foam elastomer, and/or combinations thereof.

8. The sensor of claim 1, comprising one or more adhesive layers adjacent an interior surface of the sensor (12), and configured to adhere the sensor (12) to the digit.

9. The sensor of claim 1, wherein the sensor (12) comprises a pulse oximetry sensor or a sensor for measuring a water fraction.

10. The sensor of claim 1, comprising a ribbon cable coupled to the emitter (26) and the detector (28) and capable of transmitting signals between the sensor (12) and a monitor.

11. The sensor of claim 1, wherein the body (68) comprises a generally semi-rigid molded structure comprising a generally tubular interior and the generally lengthwise opening configured to enable expansion of the generally tubular interior to accommodate a digit.

12. The sensor of claim 11, wherein the body (68) comprises an opening at an end of the semi-rigid molded structure configured to receive the digit.

13. The sensor of claim 11, wherein the body (68) comprises an opening at an end of the semi-rigid molded structure configured to enable protrusion by at least a portion of the digit.

14. The sensor of claim 1, wherein the body (68) comprises a generally flexible semi-rigid structure capable of being affixed to sensing components, and to secure the sensing components to a digit.

15. The sensor of claim 14, wherein the generally flexible semi-rigid structure comprises a plurality of wings configured to enable expansion of the sensor body (68) to accommodate the digit.

16. The sensor of claim 4, wherein the generally flexible semi-rigid structure comprises an opening configured to enable a nail of the digit to protrude therethrough.

17. A method of manufacturing a sensor (12), comprising overmolding a generally flexible semi-rigid sensor body (68) around a sensing component assembly (66) comprising an emitter (26),a detector (28) and a casing (84) enclosing the emitter (26) and the detector (28), the casing having a top (86), a bottom (88) and a lateral raised ridge (90) between the emitter (26) and the detector (28), and the method further comprises configuring the sensor (12) so that the emitter (26) and the detector (28) are at a substantially fixed optical distance relative to one another, wherein the body (68) comprises a plurality of wings (74) and the body (68) has a generally tubular interior, wherein the wings (74) are separated by an opening extending generally lengthwise along the sensor and the sensor is closed opposite the generally lengthwise opening.

## Patentansprüche

1. Sensor (12), der Folgendes umfasst:
einen Emitter (26) und einen Detektor (28), wobei sich der Emitter (26) und der Detektor (28) bei einer im Wesentlichen festgelegten optischen Entfernung im Verhältnis zueinander befinden, und
einen im Allgemeinen ausdehnbaren geformten Korpus (68), der dafür konfiguriert ist, den Emitter (26) und den Detektor (28) an einem Finger zu befestigen, **dadurch gekennzeichnet, dass**:
der Korpus (68) mehrere Flügel (74) umfasst und eine im Allgemeinen röhrenförmige Gestalt und ein im Allgemeinen röhrenförmiges Inneres hat, wobei die Flügel (74) durch eine Öffnung getrennt sind, die sich im Allgemeinen in Längsrichtung entlang des Sensors erstreckt,
der Sensor ferner eine Abfühlkomponenten-Baugruppe (66) umfasst, die dafür konfiguriert ist, an den Korpus (68) gekoppelt zu werden, und den Emitter und den Detektor umfasst und ein Gehäuse (84) mit einem Oberteil (86), einem Unterteil (88) und einer seitlichen erhöhten Leiste (90), die sich zwischen dem Emitter (26) und dem Detektor (28) befindet und die sich gegenüber der im Allgemeinen in Längsrichtung verlaufenden Öffnung befindet, wenn die Abfühlkomponenten-Baugruppe (66) und der Korpus (68) zusammengekoppelt sind.

2. Sensor nach Anspruch 1, wobei der ausdehnbare geformte Korpus (68) auf den Emitter (26) und den Detektor (28) überformt ist.

3. Sensor nach Anspruch 1, der ein im Wesentlichen starres Gehäuse (84) umfasst, das dafür konfiguriert ist, den Emitter (26) und den Detektor (28) unterzubringen.

4. Sensor nach Anspruch 3, wobei der ausdehnbare geformte Korpus (68) eine überformte Komponente ist, die dazu in der Lage ist, an dem im Wesentlichen starren Gehäuse (84) befestigt zu werden.

5. Sensor nach Anspruch 3, wobei der ausdehnbare geformte Korpus (68) eine vorgeformte Komponente ist, die dazu in der Lage ist, an dem im Wesentlichen starren Gehäuse (84) befestigt zu werden, und wahlweise die vorgeformte Komponente dazu in der Lage ist, abnehmbar an dem im Wesentlichen starren Gehäuse (84) befestigt zu werden.

6. Sensor nach Anspruch 1, wobei der ausdehnbare geformte Korpus (68) ein Elastomer mit einer Durometerhärte von etwa 15 bis etwa 30 Shore A umfasst.

7. Sensor nach Anspruch 1, wobei der ausdehnbare geformte Korpus (68) ein Polyurethan, ein Silikon-Duroplast, ein Elastomer medizinischer Güte, ein geschlossenzelliges Schaumstoff-Elastomer und/oder Kombinationen derselben umfasst.

8. Sensor nach Anspruch 1, der eine oder mehrere Klebstoffschichten angrenzend an eine Innenfläche des Sensors (12) umfasst und dafür konfiguriert ist, den Sensor (12) an den Finger zu kleben.

9. Sensor nach Anspruch 1, wobei der Sensor (12) einen Pulsoximetrie-Sensor oder einen Sensor zum Messen eines Wasseranteils umfasst.

10. Sensor nach Anspruch 1, der ein Bandkabel umfasst, das an den Emitter (26) und den Detektor (28) gekoppelt und dazu in der Lage ist, Signale zwischen dem Sensor (12) und einem Monitor zu übertragen.

11. Sensor nach Anspruch 1, wobei der Korpus (68) eine im Allgemeinen halbstarre geformte Struktur umfasst, die ein im Allgemeinen röhrenförmiges Inneres umfasst, und die im Allgemeinen in Längsrichtung verlaufende Öffnung dafür konfiguriert ist, eine Ausdehnung des im Allgemeinen röhrenförmigen Inneren zu ermöglichen, um einen Finger aufzunehmen.

12. Sensor nach Anspruch 11, wobei der Korpus (68) eine Öffnung an einem Ende der halbstarren geformten Struktur umfasst, die dafür konfiguriert ist, den Finger aufzunehmen.

13. Sensor nach Anspruch 11, wobei der Korpus (68) eine Öffnung an einem Ende der halbstarren geformten Struktur umfasst, die dafür konfiguriert ist, ein Vorspringen durch wenigstens einen Abschnitt des Fingers zu ermöglichen.

14. Sensor nach Anspruch 1, wobei der Korpus (68) eine im Allgemeinen flexible halbstarre Struktur umfasst, die dazu in der Lage ist, an Abfühlkomponenten befestigt zu werden und die Abfühlkomponenten an einem Finger zu befestigen.

15. Sensor nach Anspruch 14, wobei die im Allgemeinen flexible halbstarre Struktur mehrere Flügel umfasst, die dafür konfiguriert sind, eine Ausdehnung des Sensorkorpus (68) zu ermöglichen, um den Finger aufzunehmen.

16. Sensor nach Anspruch 4, wobei die im Allgemeinen flexible halbstarre Struktur eine Öffnung umfasst, die dafür konfiguriert ist, zu ermöglichen, dass ein Nagel des Fingers durch dieselbe vorspringt.

17. Verfahren zum Fertigen eines Sensors (12), welches das Überformen eines im Allgemeinen flexiblen halbstarren Sensorkorpus (68) um eine Abfühlkomponenten-Baugruppe (66) umfasst, die einen Emitter (26), einen Detektor (28) und ein Gehäuse (84), das den Emitter (26) und den Detektor (28) umschließt, umfasst, wobei das Gehäuse einen Oberteil (86), einen Unterteil (88) und eine seitliche erhöhte Leiste (90) zwischen dem Emitter (26) und dem Detektor (28) hat und das Verfahren ferner das Konfigurieren des Sensors (12) umfasst, so dass sich der Emitter (26) und der Detektor (28) bei einer im Wesentlichen festgelegten optischen Entfernung im Verhältnis zueinander befinden, wobei der Korpus (68) mehrere Flügel (74) umfasst und der Korpus (68) ein im Allgemeinen röhrenförmiges Inneres hat, wobei die Flügel (74) durch eine Öffnung getrennt sind, die sich im Allgemeinen in Längsrichtung entlang des Sensors erstreckt, und der Sensor gegenüber der im Allgemeinen in Längsrichtung verlaufenden Öffnung geschlossen ist.

## Revendications

1. Capteur (12), comprenant :
un émetteur (26) et un détecteur (28), l'émetteur (26) et le détecteur (28) étant situés à une distance optique essentiellement fixe l'un par rapport à l'autre ; et
un corps moulé (68) généralement extensible, configuré de sorte à fixer l'émetteur (26) et le détecteur (28) sur un doigt, **caractérisé en ce que** :
le corps (68) comprend plusieurs ailes (74) et a une forme généralement tubulaire et un intérieur généralement tubulaire, dans lequel les ailes (74) sont séparées par une ouverture s'étendant généralement longitudinalement le long du capteur ;
le capteur comprend en outre un assemblage de composants de détection (66), configuré de sorte à être accouplé audit corps (68), et comprenant ledit émetteur et ledit détecteur, et un boîtier (84) avec une partie supérieure (86), une partie inférieure (88) et une nervure latérale surélevée (90) agencée entre l'émetteur (26) et le détecteur (28) et opposée à l'ouverture généralement longitudinale lorsque l'assemblage de composants de détection (66) et le corps (68) sont accouplés.

2. Capteur selon la revendication 1, dans lequel le corps (68) moulé extensible est surmoulé sur l'émetteur (26) et le détecteur (28).

3. Capteur selon la revendication 1, comprenant un boîtier (84) essentiellement rigide, configuré de sorte à recevoir l'émetteur (26) et le détecteur (28).

4. Capteur selon la revendication 3, dans lequel le corps (68) moulé extensible est un composant surmoulé, pouvant être fixé sur le boîtier (84) essentiellement rigide.

5. Capteur selon la revendication 3, dans lequel le corps (68) moulé extensible est un composant prémoulé, pouvant être fixé sur le boîtier (84) essentiellement rigide, le composant prémoulé pouvant optionnellement être fixé de manière amovible sur le boîtier (84) essentiellement rigide.

6. Capteur selon la revendication 1, dans lequel le corps (68) moulé extensible comprend un élastomère avec une valeur au duromètre comprise entre environ 15 et environ 30 Shore A.

7. Capteur selon la revendication 1, dans lequel le corps (68) moulé extensible comprend un polyuréthane, une silicone thermodurcissable, un élastomère de qualité médicale, un élastomère à alvéoles fermés, et/ou des combinaisons de ces substances.

8. Capteur selon la revendication 1, comprenant une ou plusieurs couches adhésives adjacentes à une surface interne du capteur (12) et configurées de sorte à faire adhérer le capteur (12) sur le doigt.

9. Capteur selon la revendication 1, dans lequel le capteur (12) comprend un capteur d'oxymétrie pulsée ou un capteur pour mesurer une fraction aqueuse.

10. Capteur selon la revendication 1, comprenant un câble-ruban accouplé à l'émetteur (26) et au détecteur (28) et capable de transmettre des signaux entre le capteur (12) et un moniteur.

11. Capteur selon la revendication 1, dans lequel le corps (68) comprend une structure moulée généralement semi-rigide, comprenant un intérieur généralement tubulaire et l'ouverture généralement longitudinale, configurée de sorte à permettre l'extension de l'intérieur généralement tubulaire pour recevoir un doigt.

12. Capteur selon la revendication 11, dans lequel le corps (68) comprend une ouverture au niveau d'une extrémité de la structure moulée semi-rigide, configurée de sorte à recevoir le doigt.

13. Capteur selon la revendication 11, dans lequel le corps (68) comprend une ouverture au niveau d'une extrémité de la structure moulée semi-rigide, configurée de sorte à permettre de recevoir au moins une partie du doigt.

14. Capteur selon la revendication 1, dans lequel le corps (68) comprend une structure semi-rigide généralement flexible, pouvant être fixée sur les composants de détection et fixer les composants de détection sur un doigt.

15. Capteur selon la revendication 14, dans lequel la structure semi-rigide généralement flexible comprend plusieurs ailes, configurées de sorte à permettre l'extension du corps du capteur (68) afin de recevoir le doigt.

16. Capteur selon la revendication 4, dans lequel la structure semi-rigide généralement flexible comprend une ouverture, configurée de sorte à permettre le débordement d'un ongle du doigt à travers elle.

17. Procédé de fabrication d'un capteur (12), comprenant l'étape de surmoulage d'un corps (68) de capteur semi-rigide généralement flexible autour d'un assemblage de composants de détection (66), comprenant un émetteur (26), un détecteur (28) et un boîtier (84) renfermant l'émetteur (26) et le détecteur (28), le boîtier comportant une partie supérieure (86), une partie inférieure (88) et une nervure latérale surélevée (90) entre l'émetteur (26) et le détecteur (28), le procédé comprenant en outre l'étape de configuration du capteur (12) de sorte que l'émetteur (26) et le détecteur (28) se situent à une distance optique essentiellement fixe l'un par rapport à l'autre, dans lequel le corps (68) comprend plusieurs ailes (74), le corps (68) ayant un intérieur généralement tubulaire, les ailes (74) étant séparées par une ouverture s'étendant en général longitudinalement le long du capteur et le capteur étant fermé en un point opposé à l'ouverture généralement longitudinale.
